# FASCICULE DE BREVET EUROPEEN

(11) **EP 1 463 469 B1**
(45) Date de publication et mention de la délivrance du brevet: **24.10.2007**
(21) Numéro de dépôt: 03729274.5
(22) Date de dépôt: 08.01.2003
(51) Int. Cl.: A61F 5/00

(54) **ANNEAU GASTRIQUE DE TRAITEMENT DE L'OBESITE**
MAGENRING ZUR BEHANDLUNG VON FETTSUCHT
GASTRIC RING FOR THE TREATMENT OF OBESITY

(30) Priorité: 09.01.2002 FR 0200260
(43) Date de publication de la demande: 06.10.2004
(73) Titulaire: SOFRADIM PRODUCTION, 01600 Trévoux (FR)
(72) Inventeur: JARSAILLON, Philippe, F-42720 Pouilly sous Charlieu (FR); THERIN, Michel, F-69004 Lyon (FR)
(74) Mandataire: Brédeville, Odile Marie
(86) Numéro de dépôt international: PCT/FR2003/000038
(87) Numéro de publication internationale: WO 2003/057090

(56) Documents cités:
- EP-A- 0 611 561
- WO-A-00/41649
- FR-A- 2 612 392

## Description

La présente invention concerne un anneau gastrique de traitement de l'obésité. Un tel anneau est également couramment dénommé "anneau de gastroplastie".

Il est connu de traiter l'obésité pathologique d'un patient en plaçant un anneau autour de l'estomac de ce patient, de manière à créer, sur la partie supérieure de l'estomac, une poche de dimensions restreintes et une ouverture d'écoulement des aliments ayant également des dimensions restreintes.

De tels anneaux sont bien connus dans leur principe, et les documents WO-A-86104498 ou EP-A-0 611 561 peuvent être cités en tant que documents illustrant des anneaux gastriques existants.

Un anneau gastrique existant comprend une poche gonflable située sur sa face interne, permettant d'ajuster la section de l'ouverture délimitée par l'anneau. En effet, l'implantation d'un tel anneau, de par la dissection préalable qu'elle suppose, est plus ou moins traumatisante pour la paroi gastrique, et il convient de ne pas serrer immédiatement cet anneau autour de cette paroi afin que cette dernière puisse cicatriser.

Le gonflage de la poche est réalisé au moyen d'un fluide alimenté, par voie percutanée, à partir d'une chambre implantable et d'une conduite reliant cette chambre à la poche.

Certains des anneaux gastriques selon la technique antérieure ont pour inconvénient de rester relativement agressifs pour la paroi de l'estomac, au point de provoquer des inflammations de cette paroi, voire, dans des cas extrêmes, des perforations de celle-ci. Cette agressivité résulte notamment de la pression exercée par la poche gonflable et d'une lumière formée non parfaitement continue.

De plus, ladite chambre implantable et ladite tubulure reliant cette chambre et la poche ont pour inconvénient de présenter des risques de fuite, de migration et d'infection.

La chambre peut être plus ou moins visible sous la peau, ce qui n'est pas très favorable d'un point de vue esthétique.

La présente invention vise à remédier à l'ensemble de ces inconvénients des anneaux existants.

L'anneau qu'elle concerne comprend, de manière connue en soi,
- une bande propre à entourer la paroi de l'estomac;
- des moyens de maintien de la bande sous la forme d'un anneau propre à entourer l'estomac, et
- des moyens permettant, après implantation, de modifier la section de l'ouverture délimitée par l'anneau.

Selon l'invention,
- la bande comprend au moins une zone en un matériau déformable élastiquement dans le sens longitudinal de cette bande ;
- ladite zone présente au moins deux points d'appui aménagés sur elle en deux emplacements séparés dans le sens longitudinal de la bande, et
- l'anneau comprend au moins un élément rigide en un matériau biorésorbable ou biodégradable, prenant appui contre ladite zone au niveau desdits points d'appui, cet élément rigide permettant, avant résorption, de maintenir lesdits points d'appui à une distance l'un de l'autre différente de la distance séparant ces deux points d'appui en l'absence de déformation élastique de ladite zone de la bande, et, après résorption, ne faisant plus obstacle au retour de ladite zone de la bande dans son état de non-déformation.

Par " biodégradable" ou "biorésorbable", on entend la propriété selon laquelle un matériau se dégrade in vivo par un mécanisme cellulaire, enzymatique ou microbien (cf par exemple dégradation du collagène par la collagènase), ou par un mécanisme physico-chimique (cf par exemple hydrolyse d'un polymère d'acide lactique).

Un tel matériau biorésorbable est de préférence choisi dans le groupe consistant en les polymères de p-dioxanone, les polyglycolides, les polyorthoesters, les polymères de triméthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides. De manière encore plus préférée, le matériau biorésorbable présente une polydispersité inférieure à 2.

A titre d'exemple préféré, le matériau biodégradable ou biorésorbable est un polymère d'acide lactique (PLA) ou polyglycolique (PGA), ou un copolymère d'acide lactique ou polyglycolique (PLA-PGA).

L'anneau selon l'invention est mis en place autour de l'estomac, puis la résorption du ou des éléments en matériau biorésorbable qu'il comprend permet à ladite ou auxdites zones déformables de la bande de retrouver leur forme neutre, de non-déformation. La circonférence de l'anneau, et donc la section de l'ouverture délimitée par cet anneau, peut ainsi être adaptée aux besoins du traitement du patient.

Cet anneau ne comprend donc pas de poche sur sa face interne, ni de chambre implantable et de tubulure reliant cette chambre à cette poche. Il en résulte que l'anneau est peu agressif pour la paroi gastrique et qu'il permet d'éliminer tous les inconvénients précités liés à l'utilisation de ces poche gonflable, chambre implantable et tubulure.

Chaque élément en matériau biorésorbable est en priorité destiné à maintenir la zone de la bande le long de laquelle il s'étend dans un état d'étirement ; l'anneau peut ainsi être mis en place dans un état de relative distension pour permettre la cicatrisation de la paroi gastrique puis, après résorption de cet ou de ces éléments en matériau biorésorbable, faire l'objet d'une constriction résultant du rappel élastique du matériau constituant ladite zone déformable.

Les principes de l'invention pourraient toutefois être appliqués de manière inverse, pour l'obtention d'une distension après résorption, auquel cas chaque élément en matériau biorésorbable est destiné à maintenir la zone de la bande le long de laquelle il s'étend dans un état de constriction.

De préférence, la bande forme, à une extrémité, un oeillet délimitant un épaulement, et présente, à son autre extrémité, une forme effilée facilitant son insertion dans l'oeillet; cette autre extrémité comprend au moins un cran d'encliquetage destiné à être encliqueté dans l'oeillet et à coopérer avec l'épaulement pour maintenir la bande sous forme d'un anneau.

Ladite autre extrémité peut comprendre plusieurs crans successifs, permettant de fermer la bande selon plusieurs diamètres adaptés aux spécificités du patient à traiter.

Selon une forme possible de réalisation de l'invention,
- chaque paire de points d'appui est formée par deux zones opposées d'une ouverture aménagée radialement et au travers de ladite zone déformable de la bande, cette ouverture étant de forme circulaire lorsque la zone déformable est dans un état de non-déformation mais pouvant acquérir une forme oblongue lorsque cette zone déformable est étirée longitudinalement; et
- chaque élément rigide présente une forme tubulaire oblongue et est dimensionné de manière à pouvoir être introduit en force dans l'ouverture précitée afin de conférer à cette ouverture une forme oblongue correspondante, cette forme oblongue produisant un étirement longitudinal corrélatif de ladite zone déformable.

Chaque insert peut présenter une forme légèrement évasée, et être mis en place sur ladite zone déformable, de telle sorte que son extrémité de plus forte section soit disposée sur l'extérieur de l'anneau.

Cette forme légèrement évasée permet une adaptation de l'insert à la courbure de l'anneau et donc d'éviter tout risque d'expulsion de cet insert lorsque la bande est courbée pour former l'anneau.

Selon une autre forme possible de réalisation de l'invention,
- chaque paire de points d'appui est formée par deux trous aménagés dans ladite zone déformable de la bande, et
- chaque élément rigide présente une forme d'agrafe, c'est-à-dire comprend un corps comportant deux ergots, ces ergots étant destinés à être reçus dans lesdits trous ; la distance séparant les ergots est supérieure à la distance séparant, à l'état non étiré de ladite zone, les deux trous, de sorte que la portion de cette zone située entre ces trous est étirée lorsque ces ergots sont engagés dans ces trous.

Le corps de chaque élément selon cette autre forme de réalisation peut présenter une forme courbe, permettant l'adaptation de cet élément à la courbure de l'anneau.

Selon encore une autre forme possible de réalisation de l'invention,
- chaque paire de points d'appui est formée par deux épaulements délimités par une portion de section transversale moindre que celle que présente ladite zone déformable, et
- chaque élément rigide présente une forme tubulaire et est destiné à être engagé sur cette portion de section transversale moindre, de manière à prendre appui contre les épaulements, la longueur de cet élément étant supérieure à la distance séparant, à l'état non étiré de ladite zone déformable, lesdits épaulements, de sorte que la venue des extrémités de l'élément tubulaire contre ces épaulements réalise un étirement de ladite portion de section transversale moindre.

L'élément peut présenter une forme courbe correspondant à la courbure de l'anneau, également pour permettre l'adaptation de cet élément à la courbure de l'anneau.

L'invention sera bien comprise à l'aide de la description qui suit, faite en référence au dessin schématique annexé, qui représente, à titre d'exemples non limitatifs, trois formes de réalisation possibles de l'anneau gastrique concerné.

Les figures 1, 3 et 4 sont des vues de cet anneau selon respectivement ces trois formes de réalisation, en coupe passant par le plan médian de l'épaisseur de cet anneau, et
la figure 2 est une vue partielle de l'anneau montré par la figure 1, selon la direction de la flèche A de cette figure.

Par simplification, les éléments ou parties d'éléments qui se retrouvent d'une forme de réalisation à une autre sont désignés par les mêmes références numériques.

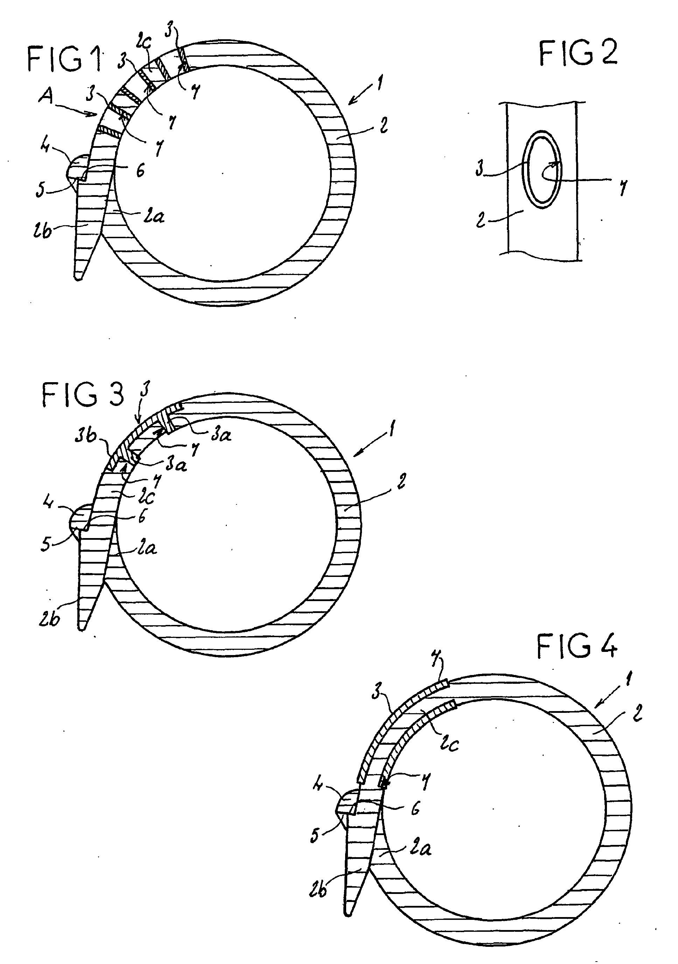

La figure 1 représente un anneau gastrique 1 de traitement de l'obésité pathologique d'un patient, couramment dénommé "anneau de gastroplastie".

L'anneau 1 comprend une bande 2 propre à entourer la paroi de l'estomac, et trois inserts rigides 3.

La bande 2 est en un matériau déformable élastiquement dans le sens longitudinal de cette bande, et est notamment en silicone. A une extrémité 2a, elle forme un oeillet 4 délimitant un épaulement 5. A son autre extrémité 2b, elle présente une forme effilée facilitant son insertion dans l'oeillet 4 et comprend un cran d'encliquetage 6 destiné à être encliqueté dans l'oeillet 4 et à coopérer avec l'épaulement 5 pour maintenir la bande 2 sous forme d'un anneau. Cette extrémité 2b peut comprendre plusieurs crans 6 successifs, permettant de fermer la bande 2 selon plusieurs diamètres adaptés aux spécificités du patient à traiter.

Du côté de son extrémité 2b, la bande 2 présente une zone 2c dans laquelle sont aménagés trois ouvertures radiales traversantes 7. Chaque ouverture 7 est circulaire lorsque la bande 2 est dans un état de non-déformation mais peut acquérir une forme oblongue lorsque cette zone 2c est étirée longitudinalement.

Chaque insert 3 est en un matériau biorésorbable ou biodégradable tel qu'un polymère d'acide lactique (PLA) ou polyglycolique (PGA), ou un copolymère d'acide lactique ou polyglycolique (PLA-PGA). Comme le montre la figure 2, il présente une forme tubulaire oblongue et est dimensionné de manière à pouvoir être introduit en force dans une ouverture 7. Il confère ainsi à cette ouverture 7 une forme oblongue correspondante produisant un étirement longitudinal corrélatif de la zone 2c.

Pour s'adapter à la courbure que présente la bande 2 lorsque les extrémités 2a et 2b sont en prise, les inserts 3 présentent une forme légèrement évasée, leurs extrémités de plus forte section étant disposées sur l'extérieur de l'anneau 1.

En pratique, la bande 2 est introduite dans le corps du patient et est mise en place autour de l'estomac, par une technique de pose peu invasive telle que laparoscopie, puis l'extrémité 2b est engagée au travers de l'oeillet 4 jusqu'à encliquetage du cran 6 au-delà de l'oeillet 4 et venue de ce cran 6 en appui contre l'épaulement 5.

Les emplacements respectifs de ce cran 6 et de cet épaulement 5 ont été prédéterminés de telle sorte que l'anneau 1 ainsi formé présente une relative distension par rapport au serrage souhaité de la paroi gastrique, afin de permettre la cicatrisation de la paroi gastrique avant d'exercer une contrainte de serrage sur cette paroi.

L'épaisseur des inserts 3 est calculée, en fonction du matériau utilisé, de telle sorte que se produise, sous environ un à deux mois après l'implantation, une rupture mécanique de ces inserts 3, cette rupture se faisant sous l'effet combiné de la résorption des inserts 3 et de la tension exercée par la bande 2. La rupture de ces inserts 3 permet à la zone 2c de retrouver sa forme neutre, de non-étirement.

L'anneau 1 fait alors l'objet d'une constriction résultant du rappel élastique du matériau constituant ladite zone 2c, qui donne à la circonférence de l'anneau 1, et donc à la section de l'ouverture délimitée par cet anneau 1, des dimensions adaptées aux besoins du traitement du patient.

Dans le cas de l'anneau 1 montré sur la figure 3, la zone 2c comprend deux trous 7 qui reçoivent les ergots 3a d'un élément inséré 3 en forme d"'agrafe". La distance séparant les ergots 3a est supérieure à la distance séparant, à l'état non étiré de la zone 2c, les deux trous 7, de sorte que la portion de cette zone 2c située entre ces trous 7 est étirée lorsque ces ergots 3a sont engagés dans ces trous 7.

Le corps 3b de l'élément 3 qui comporte les ergots 3a présente une forme courbe, permettant l'adaptation de cet élément 3 à la courbure de l'anneau 1.

Dans le cas de l'anneau 1 montré sur la figure 4, la zone 2c présente une portion de section transversale moindre formant deux épaulements 7. Un élément tubulaire 3 est engagé sur cette portion de section transversale moindre et prend appui contre ces épaulements 7. La longueur de l'élément 3 est supérieure à la distance séparant, à l'état non étiré de la zone 2c, les épaulements 7, de sorte que la venue des extrémités de l'élément 3 contre les épaulements 7 réalise un étirement de ladite portion de section transversale moindre.

Ici également, l'élément 3 présente une forme courbe correspondant à la courbure de l'anneau 1.

Cet anneau gastrique 1 apporte une amélioration déterminante à la technique antérieure, étant donné qu'il ne comprend pas de poche gonflable sur sa face interne, ni de chambre implantable et de tubulure reliant cette chambre à cette poche. II en résulte que cet anneau est peu agressif pour la paroi gastrique et qu'il permet d'éliminer tous les inconvénients liés à l'utilisation de ces poche gonflable, chambre implantable et tubulure.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, l'anneau peut comprendre plusieurs zones déformables 2c ; la bande 2 peut être à structure monolithique comme représenté ou peut avoir, en dehors de la ou des zones 2c, une structure différente de celle de cette ou ces zones 2c; chaque élément 3 est en priorité destiné à maintenir la zone 2c correspondante dans un état d'étirement, mais chaque zone 2c pourrait être maintenue dans un état de constriction par un ou plusieurs de ces éléments 3, pour l'obtention d'une distension de l'anneau après résorption de ces éléments 3 ; l'élément 3 peut se présenter sous la forme d'une "agrafe" comme représenté sur la figure 3 mais avec des ergots 3a ayant une longueur telle qu'ils traversent ladite zone déformable 2c, et recevant une plaquette engagée sur eux et les reliant l'un à l'autre, cette plaquette permettant de répartir les efforts exercés sur ces ergots 3a ; ces ergots 3a et cette plaquette peuvent être conformés de manière à réaliser un assemblage, notamment par encliquetage, entre ces ergots 3a et cette plaquette ; pour ne pas former de surépaisseur sur la face interne de l'anneau, les trous de la zone déformable 2c destinés à recevoir lesdits ergots 3a peuvent être aménagés sensiblement perpendiculairement à la zone 2c, de manière à se trouver sensiblement parallèles à l'axe de l'anneau 1 après formation de ce dernier, de sorte que le corps 3b reliant ces ergots 3a se trouve sur un côté axial de l'anneau 1 et ladite plaquette sur l'autre côté axial de cet anneau 1.

## Revendications

1. - Anneau gastrique de traitement de l'obésité, comprenant :
- une bande (2) propre à entourer la paroi de l'estomac;
- des moyens (4, 5, 6) de maintien de la bande (2) sous la forme d'un anneau propre à entourer l'estomac, et
- des moyens (3, 7) permettant, après implantation, de modifier la section de l'ouverture délimitée par l'anneau (1);
anneau gastrique (1) **caractérisé en ce que** :
- la bande (2) comprend au moins une zone (2c) en un matériau déformable élastiquement dans le sens longitudinal de cette bande (2) ;
- ladite zone (2c) présente au moins deux points d'appui (7) aménagés sur elle en deux emplacements séparés dans le sens longitudinal de la bande (2), et
- l'anneau (1) comprend au moins un élément rigide (3) en un matériau biorésorbable ou biodégradable, prenant appui contre ladite zone (2c) au niveau desdits points d'appui (7), cet élément rigide (3) permettant, avant résorption, de maintenir lesdits points d'appui (7) à une distance l'un de l'autre différente de la distance séparant ces deux points d'appui (7) en l'absence de déformation élastique de ladite zone (2c) de la bande (2), et, après résorption, ne faisant plus obstacle au retour de ladite zone (2c) de la bande (2) dans son état de non-déformation.

2. - Anneau gastrique selon la revendication 1, **caractérisé en ce que** chaque élément (3) en matériau biorésorbable est conformé de manière à maintenir la zone (2c) de la bande (2) le long de laquelle il s'étend dans un état d'étirement.

3. - Anneau gastrique selon la revendication 1, **caractérisé en ce que** chaque élément (3) en matériau biorésorbable est conformé de manière à maintenir la zone (2c) de la bande (2) le long de laquelle il s'étend dans un état de constriction.

4. - Anneau gastrique selon l'une des revendications 1 à 3, **caractérisé en ce que** la bande (2) forme, à une extrémité (2a), un oeillet (4) délimitant un épaulement (5), et **en ce qu'**elle présente, à son autre extrémité (2b), une forme effilée facilitant son insertion dans l'oeillet (4), cette autre extrémité (2b) comprenant au moins un cran d'encliquetage (6) destiné à être encliqueté dans l'oeillet (4) et à coopérer avec l'épaulement (5) pour maintenir la bande (2) sous forme d'un anneau.

5. - Anneau gastrique selon la revendication 4, **caractérisé en ce que** ladite autre extrémité (2b) comprend plusieurs crans (6) successifs, permettant de fermer la bande (2) selon plusieurs diamètres adaptés aux spécificités du patient à traiter.

6. - Anneau gastrique selon l'une des revendications 1 à 5, **caractérisé :**
- **en ce que** chaque paire de points d'appui est formée par deux zones opposées d'une ouverture (7) aménagée radialement et au travers de ladite zone déformable (2c) de la bande (2), cette ouverture (7) étant de forme circulaire lorsque la zone déformable (2c) est dans un état de non-déformation mais pouvant acquérir une forme oblongue lorsque cette zone déformable (2c) est étirée longitudinalement ; et
- **en ce que** chaque élément rigide (3) présente une forme tubulaire oblongue et est dimensionné de manière à pouvoir être introduit en force dans l'ouverture (7) précitée afin de conférer à cette ouverture (7) une forme oblongue correspondante, cette forme oblongue produisant un étirement longitudinal corrélatif de ladite zone déformable (2c).

7. - Anneau gastrique selon la revendication 6, **caractérisé en ce que** chaque insert (3) présente une forme légèrement évasée, et est destiné à être mis en place sur ladite zone déformable (2c) de telle sorte que son extrémité de plus forte section soit disposée sur l'extérieur de l'anneau (1).

8. - Anneau gastrique selon l'une des revendications 1 à 5, **caractérisé en ce que** :
- chaque paire de points d'appui est formée par deux trous (7) aménagés dans ladite zone déformable (2c) de la bande (2), et
- chaque élément rigide (3) présente une forme d'agrafe, c'est-à-dire comprend un corps (3b) comportant deux ergots (3a), ces ergots (3a) étant destinés à être reçus dans les trous (7) ; la distance séparant les ergots (3a) est supérieure à la distance séparant, à l'état non étiré de ladite zone (2c), les deux trous (7), de sorte que la portion de cette zone (2c) située entre ces trous (7) est étirée lorsque ces ergots (3a) sont engagés dans ces trous (7).

9. - Anneau gastrique selon la revendication 8, **caractérisé en ce que** le corps (3b) présente une forme courbe, permettant l'adaptation de cet élément (3) à la courbure de l'anneau (1).

10. -Anneau gastrique selon la revendication 7 ou la revendication 8, **caractérisé en ce que** les ergots (3a) présentent une longueur telle qu'ils traversent ladite zone déformable (2c) et **en ce que** ces ergots (3a) reçoivent une plaquette engagée sur eux et les reliant l'un à l'autre.

11. - Anneau gastrique selon la revendication 10, **caractérisé en ce que** les ergots (3a) et ladite plaquette sont conformés de manière à réaliser un assemblage, notamment par encliquetage, entre ces ergots (3a) et cette plaquette.

12. - Anneau gastrique selon l'une des revendications 8 à 11, **caractérisé en ce que** les trous (7) de la zone déformable (2c) destinés à recevoir lesdits ergots (3a) sont aménagés sensiblement perpendiculairement à cette zone déformable (2c), de manière à se trouver sensiblement parallèles à l'axe de l'anneau 1 après formation de ce dernier.

13. - Anneau gastrique selon l'une des revendications 1 à 5, **caractérisé en ce que** :
- chaque paire de points d'appui est formée par deux épaulements (7) délimités par une portion de section transversale moindre que celle que présente ladite zone déformable (2c), et
- chaque élément rigide (3) présente une forme tubulaire et est destiné à être engagé sur cette portion de section transversale moindre, de manière à prendre appui contre les épaulements (7), la longueur de cet élément (3) étant supérieure à la distance séparant, à l'état non étiré de ladite zone déformable (2c), lesdits épaulements (7), de sorte que la venue des extrémités de l'élément tubulaire (3) contre ces épaulements (7) réalise un étirement de ladite portion de section transversale moindre.

14. - Anneau gastrique selon la revendication 13, **caractérisé en ce que** l'élément (3) présente une forme courbe correspondant à la courbure de l'anneau (1).

15. -Anneau gastrique selon l'une des revendications 1 à 14, **caractérisé en ce qu'**au moins ladite zone déformable (2c), sinon l'ensemble de la bande (2), est en silicone.

16. - Anneau gastrique selon l'une des revendications 1 à 15, **caractérisé en ce que** chaque élément rigide (3) est en un polymère d'acide lactique ou polyglycolique, ou en un copolymère d'acide lactique ou polyglycolique.

## Claims

1. A gastric ring for treatment of obesity, comprising:
- a band (2) which is able to surround the wall of the stomach;
- means (4, 5, 6) for maintaining the band (2) in the form of a ring able to surround the stomach, and
- means (3, 7) with which it is possible, after implantation, to modify the cross section of the opening delimited by the ring (1);
said gastric ring (1) being **characterized in that**:
- the band (2) comprises at least one zone (2c) made of a material which is elastically deformable in the longitudinal direction of this band (2);
- said zone (2c) has at least two bearing points (7) formed on it at two locations separated in the longitudinal direction of the band (2), and
- the ring (1) comprises at least one rigid element (3) made of a bioabsorbable or biodegradable material and bearing against said zone (2c) in the area of said bearing points (7), this rigid element (3), before absorption, making it possible to maintain said bearing points (7) at a distance from one another different than the distance separating these two bearing points (7) in the absence of elastic deformation of said zone (2c) of the band (2), and, after absorption, no longer forming an obstacle to the return of said zone (2c) of the band (2) to its nondeformed state.

2. The gastric ring as claimed in claim 1, **characterized in that** each element (3) made of bioabsorbable material is designed in such a way as to maintain the zone (2c) of the band (2), along which it extends, in a stretched state.

3. The gastric ring as claimed in claim 1, **characterized in that** each element (3) made of bioabsorbable material is designed in such a way as to maintain the zone (2c) of the band (2), along which it extends, in a contracted state.

4. The gastric ring as claimed in one of claims 1 through 3, **characterized in that** the band (2) forms, at one end (2a), an eyelet (4) delimiting a shoulder (5), and **in that** it has, at its other end (2b), a tapered shape facilitating its insertion into the eyelet (4), this other end (2b) comprising at least one snap-fit catch (6) intended to be snap-fitted into the eyelet (4) and to cooperate with the shoulder (5) in order to maintain the band (2) in the form of a ring.

5. The gastric ring as claimed in claim 4, **characterized in that** said other end (2b) comprises several successive catches (6), making it possible to close the band (2) at several diameters adapted to the specific circumstances of the patient to be treated.

6. The gastric ring as claimed in one of claims 1 through 5, **characterized in that**:
- each pair of bearing points is formed by two opposite zones of an opening (7) formed radially and through said deformable zone (2c) of the band (2), this opening (7) being of circular shape when the deformable zone (2c) is in a nondeformed state, but being able to assume an oblong shape when this deformable zone (2c) is stretched longitudinally; and
- each rigid element (3) has an oblong tubular shape and is dimensioned so as to be able to be introduced with force into the aforementioned opening (7) in order to confer on this opening (7) a corresponding oblong shape, this oblong shape producing a correlated longitudinal stretching of said deformable zone (2c).

7. The gastric ring as claimed in claim 6, **characterized in that** each insert (3) has a slightly flared shape and is intended to be placed on said deformable zone (2c) in such a way that its end of greater cross section is disposed on the outside of the ring (1).

8. The gastric ring as claimed in one of claims 1 through 5, **characterized in that**:
- each pair of bearing points is formed by two holes (7) formed in said deformable zone (2c) of the band (2), and
- each rigid element (3) has a staple shape, that is to say comprises a body (3b) having two stubs (3a), these stubs (3a) being intended to be received in the holes (7); the distance separating the stubs (3a) is greater than the distance separating the two holes (7) when said zone (2c) is in the unstretched state, so that the portion of this zone (2c) situated between these holes (7) is stretched when these stubs (3a) are engaged in these holes (7).

9. The gastric ring as claimed in claim 8, **characterized in that** the body (3b) has a curved shape permitting adaptation of this element (3) to the curvature of the ring (1).

10. The gastric ring as claimed in claim 7 or claim 8, **characterized in that** the stubs (3a) have a length which is such that they traverse said deformable zone (2c), and **in that** these stubs (3a) receive a small plate engaged on them and connecting them to one another.

11. The gastric ring as claimed in claim 10, **characterized in that** the stubs (3a) and said small plate are designed in such a way as to form an assembly, in particular by snap-fitting, between these stubs (3a) and this small plate.

12. The gastric ring as claimed in one of claims 8 through 11, **characterized in that** the holes (7) of the deformable zone (2c) which are intended to receive said stubs (3a) are formed substantially perpendicular to this deformable zone (2c), in such a way as to be located substantially parallel to the axis of the ring (1) after formation of the latter.

13. The gastric ring as claimed in one of claims 1 through 5, **characterized in that**:
- each pair of bearing points is formed by two shoulders (7) delimited by a portion with a cross section smaller than that of said deformable zone (2c), and
- each rigid element (3) has a tubular shape and is intended to be engaged on this portion of smaller cross section, in such a way as to bear against the shoulders (7), the length of this element (3) being greater than the distance separating said shoulders (7) when said deformable zone (2c) in is the unstretched state, in such a way that, when the ends of the tubular element (3) are placed against these shoulders (7), said portion of smaller cross section is stretched.

14. The gastric ring as claimed in claim 13, **characterized in that** the element (3) has a curved shape corresponding to the curvature of the ring (1).

15. The gastric ring as claimed in one of claims 1 through 14, **characterized in that** at least said deformable zone (2c), if not the whole of the band (2), is made of silicone.

16. The gastric ring as claimed in one of claims 1 through 15, **characterized in that** each rigid element (3) is made of a lactic acid or polyglycolic acid polymer, or of a lactic acid or polyglycolic acid copolymer.

## Patentansprüche

1. Magenring zur Behandlung von Fettleibigkeit, mit:
- einem Band (2), das geeignet ist, die Magenwand zu umgeben;
- Haltemitteln (4, 5, 6) zum Halten des Bandes (2) in Form eines Ringes, der geeignet ist, den Magen zu umgeben, und
- Mitteln (3, 7), die es ermöglichen, den Querschnitt der Öffnung, die durch den Ring (1) umgrenzt ist, nach einer Implantation zu ändern;
wobei der Magenring (1) **dadurch gekennzeichnet ist, dass**
- das Band (2) zumindest einen Bereich (2c) aus einem in Längsrichtung des Bandes (2) elastisch verformbaren Material aufweist;
- der Bereich (2c) zumindest zwei Anlagepunkte (7) aufweist, die in diesem an zwei in Längsrichtung des Bandes (2) voneinander getrennten Stellen ausgebildet sind, und
- der Ring (1) zumindest ein starres Element (3) aus einem bioabsorbierbaren oder biodegradierbaren Material aufweist, das im Bereich der Anlagepunkte (7) am Bereich (2c) anliegt, wobei das starre Element (3) es ermöglicht, die Anlagepunkte (7), vor einer Resorption, in einem Abstand von einander zu halten, der anders ist als der Abstand, der die zwei Anlagepunkte (7) in Abwesenheit der elastischen Verformung des Bereiches (2c) des Bandes (2) trennt, und, nach einer Resorption, kein Hindernis mehr bei der Rückkehr des Bereichs (2c) des Bandes (2) in seinen nicht verformten Zustand darstellt.

2. Magenring nach Anspruch 1, **dadurch gekennzeichnet, dass** jedes Element (3) aus dem bioabsorbierbaren Material derart ausgebildet ist, dass es den Bereich (2c) des Bandes (2), entlang dessen es sich erstreckt, in einem gestreckten Zustand hält.

3. Magenring nach Anspruch 1, **dadurch gekennzeichnet, dass** jedes Element (3) aus dem bioabsorbierbaren Material derart ausgebildet ist, dass es den Bereich (2c) des Bandes (2), entlang dessen es sich erstreckt, in einem gestauchten Zustand hält.

4. Magenring nach einem der Ansprüche 1 bis 3, **dadurch gekennzeichnet, dass** das Band (2) an einem Ende (2a) eine Öse (4) bildet, die eine Schulter (5) begrenzt, und dass das Band (2) an seinem anderen Ende (2b) eine spitz zulaufende Form aufweist, die dessen Einführen in die Öse (4) erleichtert, wobei dieses andere Ende (2b) zumindest eine Sperraste (6) aufweist, die dazu bestimmt ist, in der Öse (4) eingerastet zu werden und mit der Schulter (5) zusammenzuwirken, um das Band (2) in Form eines Ringes zu halten.

5. Magenring nach Anspruch 4, **dadurch gekennzeichnet, dass** das andere Ende (2b) mehrere aufeinander folgende Rasten (6) aufweist, die es ermöglichen, das Band (2) in mehreren Durchmessern zu schließen, die an die spezifischen Gegebenheiten des zu behandelnden Patienten angepasst sind,

6. Magenring nach einem der Ansprüche 1 bis 5, **dadurch gekennzeichnet, dass**
- jedes Paar der Anlagepunkte durch zwei gegenüberliegende Bereiche einer Öffnung (7) gebildet ist, die radial und quer durch den verformbaren Bereich (2c) des Bandes (2) ausgebildet ist, wobei die Öffnung (7) eine Kreisform aufweist, wenn sich der verformbare Bereich (2c) in einem nicht verformten Zustand befindet, die jedoch eine längliche Form einnehmen kann, wenn der verformbare Bereich (2c) längs gestreckt ist; und
- jedes starre Element (3) eine längliche Röhrenform aufweist und derart bemessen ist, um mit Kraft in die oben genannte Öffnung (7) eingeführt werden zu können, um der Öffnung (7) eine entsprechende längliche Form zu verleihen, wobei die längliche Form ein korrelatives Längsstrecken des verformbaren Bereiches (2c) bewirkt.

7. Magenring nach Anspruch 6, **dadurch gekennzeichnet, dass** jedes Insert (3) eine sich leicht ausgeweitete Form aufweist und dazu bestimmt ist, an dem verformbaren Bereich (2c) derart angebracht zu werden, dass sein Ende mit größerem Querschnitt an der Außenseite des Ringes (1) angeordnet ist.

8. Magenring nach einem der Ansprüche 1 bis 5, **dadurch gekennzeichnet, dass**
- jedes Paar der Anlagepunkte durch zwei Löcher (7) gebildet ist, die in dem verformbaren Bereich (2c) des Bandes (2) ausgebildet sind, und
- jedes starre Element (3) eine Heftklammerform aufweist, d.h. einen Körper (3b) mit zwei Haltestiften (3a) aufweist, wobei die Haltestifte (3a) dazu bestimmt sind, in den Löchern (7) aufgenommen zu werden; wobei der Abstand, der die Haltestifte (3a) trennt, größer ist als der Abstand, der in dein nicht gestreckten Zustand des Bereiches (2c) die zwei Löcher (7) trennt, so dass der Teil des Bereichs (2c), der zwischen den Löchern (7) angeordnet ist, gestreckt ist, wenn die Haltestifte (3a) in diesen Löchern (7) eingesetzt sind.

9. Magenring nach Anspruch 8, **dadurch gekennzeichnet, dass** der Körper (3b) eine gekrümmte Form aufweist, die die Anpassung des Elements (3) an die Krümmung des Ringes (1) ermöglicht.

10. Magenring nach Anspruch 7 oder 8, **dadurch gekennzeichnet, dass** die Haltestifte (3a) eine Länge aufweisen, die derart ist, dass sie den verformbaren Bereich (2c) durchqueren, und dass die Haltestifte (3a) eine Plakette aufnehmen, die auf diesen angesetzt ist und diese miteinander verbindet.

11. Magenring nach Anspruch 10, **dadurch gekennzeichnet, dass** die Haltestifte (3a) und die Plakette derart ausgebildet sind, dass sie einen Zusammenbau, insbesondere durch Einrasten, zwischen den Haltestiften (3a) und der Plakette bilden.

12. Magenring nach einem der Ansprüche 8 bis 11, **dadurch gekennzeichnet, dass** die Löcher (7) des verformbaren Bereichs (2c), die dazu bestimmt sind, die Haltestifte (3a) aufzunehmen, im Wesentlichen senkrecht zu dem verformbaren Bereich (2c) derart ausgebildet sind, dass sie sich im Wesentlichen parallel zu der Achse des Ringes (1) nach seiner Ausbildung befinden.

13. Magenring nach einem der Ansprüche 1 bis 5, **dadurch gekennzeichnet, dass**
- jedes Paar der Anlagepunkte durch zwei Schultern (7) gebildet ist, die durch einen Teil eines Querschnitts definiert sind, wobei der Querschnitt kleiner ist als der Querschnitt des deformierbaren Bereichs (2c), und
- jedes starre Element (3) eine Röhrenform aufweist und dazu bestimmt ist, auf dem Bereich mit dem kleineren Querschnitt derart angesetzt zu werden, dass dieses sich gegen die Schultern (7) abstützt, wobei die Länge des Elements (3) größer ist als der Abstand, der in dem nicht gestreckten Zustand des verformbaren Bereichs (2c) die Schultern (7) trennt, so dass das Abstützen der Enden des rohrförmigen Elements (3) gegen die Schultern (7) das Strecken des Bereichs mit dem kleineren Querschnitt bewirkt.

14. Magenring nach Anspruch 13, **dadurch gekennzeichnet, dass** das Element (3) eine gekrümmte Form aufweist, die der Krümmung des Rings (1) entspricht.

15. Magenring nach einem der Ansprüche 1 bis 14, **dadurch gekennzeichnet, dass** zumindest der verformbare Bereich (2c), wenn nicht das ganze Band (2), aus Silikon gefertigt ist.

16. Magenring nach einem der Ansprüche 1 bis 15, **dadurch gekennzeichnet, dass** jedes starre Element (3) aus einem Milchsäurepolymer oder einem Polyglykolsäurepolymer oder aus einem Milchsäurecopolymer oder Polyglykolsäurecopolymer gefertigt ist.
